# EUROPEAN PATENT APPLICATION

(11) **EP 1 955 645 A1**
(43) Date of publication of application: **13.08.2008**
(21) Application number: 07025230.9
(22) Date of filing: 28.12.2007
(51) Int. Cl.: A61B 1/00

(54) **Actuator apparatus, image pickup apparatus and endoscopic apparatus**

(30) Priority: 09.02.2007 JP 2007031159
(71) Applicant: Olympus Medical Systems Corp., Tokyo 151-0072 (JP)
(72) Inventor: Iwasaki, Seiji, Tokyo 151-0072 (JP)
(74) Representative: Thum, Bernhard

(57) **Abstract**

An actuator apparatus 2 provided in an image pickup apparatus and an endoscopic apparatus according to the invention includes a mobile unit 4 that is movable forward and backward, an urging unit 10 that urges the mobile unit in one direction, and a wire 8 (28) fixed to the mobile unit. In this case, both ends of the wire are connected to a driving source that moves the mobile unit in the other direction against the urging force by the urging unit. According to the invention, the implementation of a small actuator apparatus in a simple structure allows the reduction of the diameter of the image pickup apparatus and endoscopic apparatus including the actuator.

## Description

### BACKGROUND OF THE INVENTION

### 1. Field of the Invention

The present invention relates to an actuator apparatus, image pickup apparatus, and endoscopic apparatus having a small diameter.

### 2. Description of the Related Art

As well known, an endoscopic apparatus has been widely used for observing and/or treating the inside of (a body cavity of) a human body or for examining and/repairing within industrial plant facilities. In recent years, an image pickup apparatus may be used which has an observation optical system to be moved in the optical axis direction for shooting to switch to a focus function and zooming/tele function to a subject.

This kind of technology that adjusts a lens frame for a focusing function, for example, in an image pickup apparatus is disclosed in Japanese Unexamined Patent Application Publication No. 5-341209. The patent document discloses a technology that moves a lens frame by fixing one end of a coil spring formed by a shape memory alloys (which will be called "SMA" hereinafter) wire to a projection integrated to the lens frame to which a lens is attached and passing or not passing a current to the coil spring through two lead lines connected thereto.

However, in the technology in the Japanese Unexamined Patent Application Publication No. 5-341209, the lens frame is moved for driving an actuator in order to place two lead lines for grounding and drive signal supply to the distal end portion and proximal end portion of the coil spring formed by an SMA material. In this case, the technology has a problem of structural complexity since the lead line connecting to the distal end portion is bent for wiring.

An SMA is incompatible with bonding by soldering or an adhesive, for example. For this reason, it is difficult to reduce the size of the connecting part between the SMA wire and the lead line, and the lead line must be bent for providing a space where the lead line is movable.

Therefore, it is difficult to reduce the size of the image pickup apparatus itself. As a result, it is difficult to reduce the size of the distal end, that is, the size of the diameter of the endoscope, which is a problem.

The invention was made in view of these situations, and it is an object of the invention to implement a small actuator apparatus in a simple structure and to reduce the diameter of an image pickup apparatus and endoscopic apparatus having the actuator apparatus.

### SUMMARY OF THE INVENTION

According to an aspect of the present invention, there is provided an actuator apparatus including a mobile unit that is movable forward or backward, an urging unit that urges the mobile unit in one direction, and a wire fixed to the mobile unit, wherein both ends of the wire are connected to a driving source that moves the mobile unit in the other direction against the urging force by the urging unit.

According to another aspect of the present invention, there is provided an image pickup apparatus including the actuator apparatus, wherein the mobile unit has an optical lens, and the wire is connected so as to fold back at a latching member connecting to the mobile unit.

According to another aspect of the present invention, there is provided an endoscopic apparatus including the image pickup apparatus, wherein a rigid portion, a bending portion and a flexible portion are connected sequentially from the distal end, and one and the other ends of the wire are connected to the cable at the front and the rear portion of the bending portion, respectively.

The above and other objects, features and advantages of the invention will become more clearly understood from the following description referring to the accompanying drawings.

### BRIEF DESCRIPTION OF THE DRAWINGS

Fig. 1 is a configuration diagram of an image pickup apparatus for an electronic endoscope according to a first embodiment;
Fig. 2 is a section diagram taken by the line II-II in Fig. 3;
Fig. 3 is a front view of the distal end portion of an endoscope;
Fig. 4 is a section diagram taken by the line IV-IV in Fig. 1;
Fig. 5 is an enlarged diagram of the part V in Fig. 4;
Fig. 6 is a configuration diagram of an insulating ball;
Fig. 7 is a configuration diagram of another insulating ball;
Fig. 8 is an enlarged diagram of a part where a lens moves;
Fig. 9 is a section diagram corresponding to Fig. 4 according to a second embodiment;
Fig. 10 is a section diagram corresponding to Fig. 4 according to a third embodiment;
Fig. 11 is a section diagram corresponding to Fig. 4 according to a fourth embodiment; and
Fig. 12 is a section diagram corresponding to Fig. 4 according to a fifth embodiment.

### DETAILED DESCRIPTION OF THE PREFERRED EMBODIMENTS

The present invention will be described based on embodiments with reference to drawings below.

### [First Embodiment]

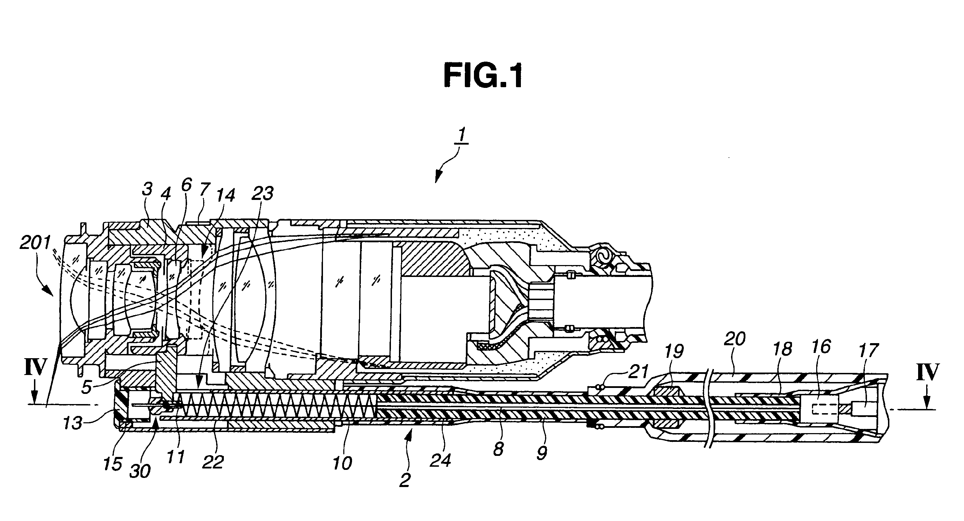

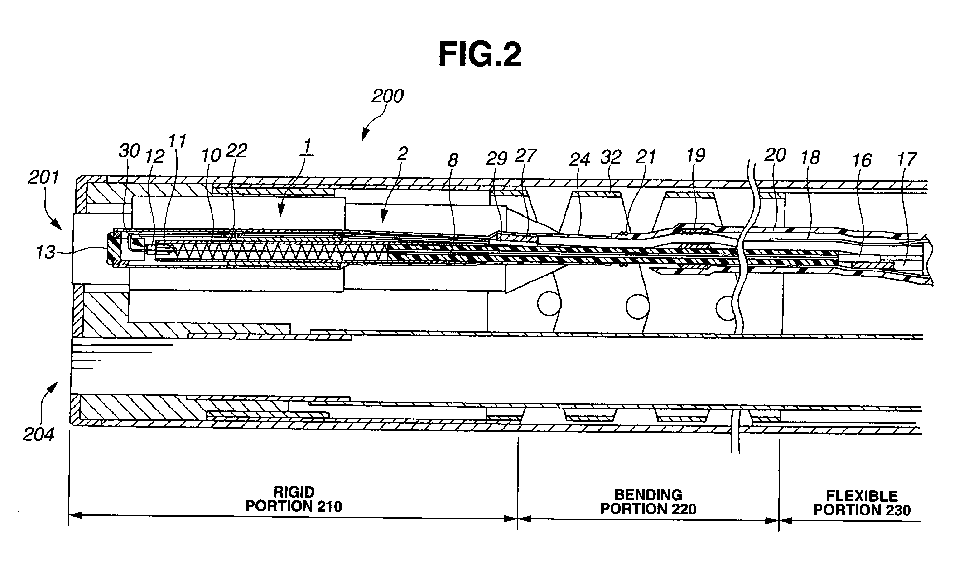

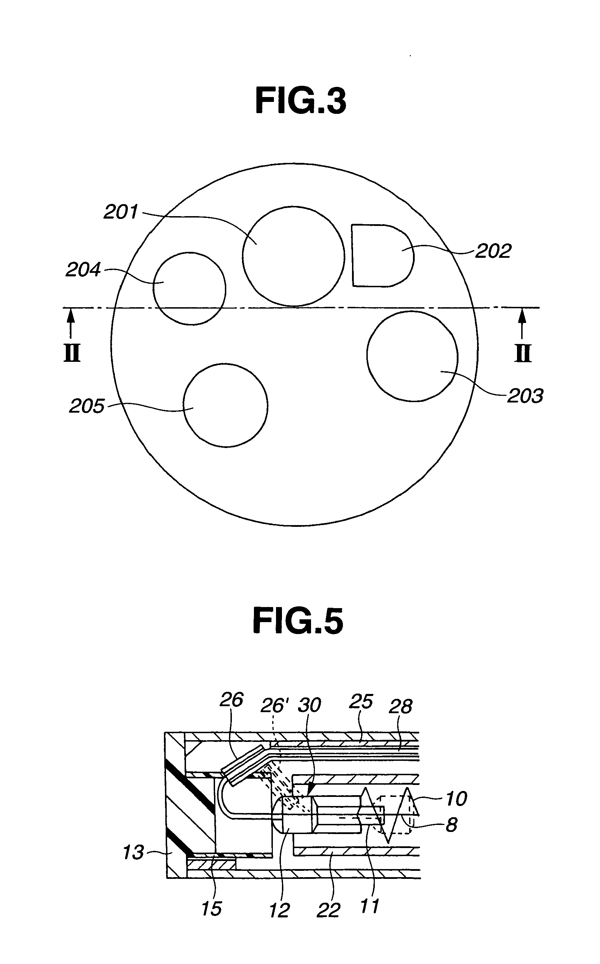

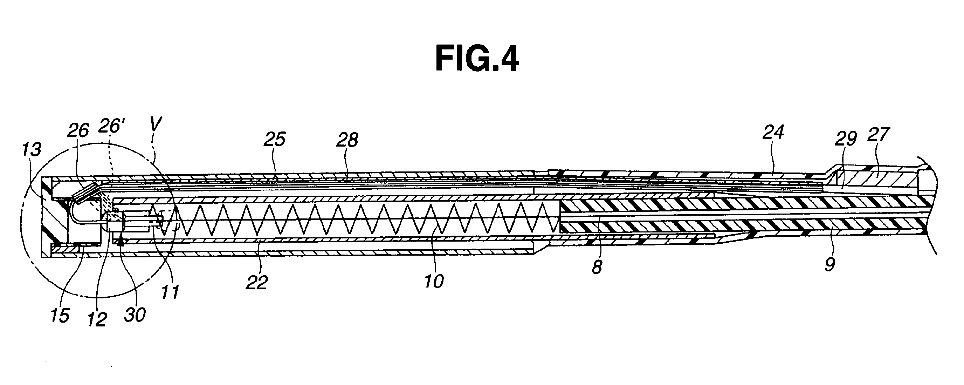

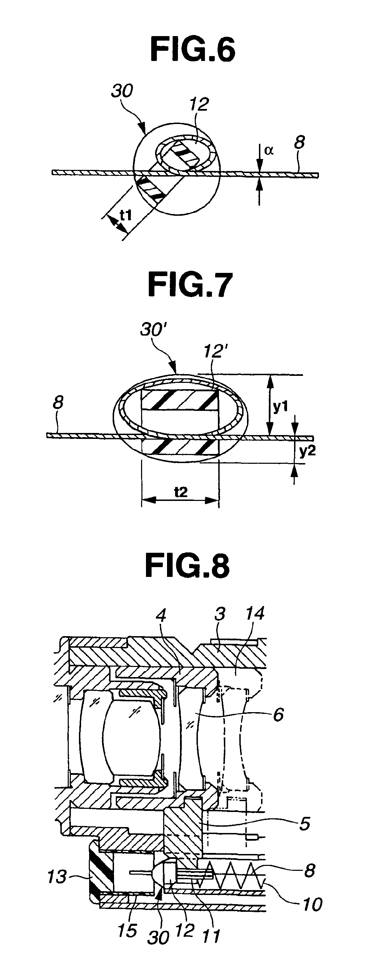

First of all, the present invention will be described with reference to Figs. 1 to 8. Figs. 1 to 8 relate to a first embodiment of the present invention, and Fig. 1 is a configuration diagram of an image pickup apparatus for an electronic endoscope, Fig. 2 is a section diagram taken by the line II-II in Fig. 3, Fig. 3 is a front view of the distal end portion of an endoscope, Fig. 4 is a section diagram taken by the line IV-IV in Fig. 1, Fig. 5 is an enlarged diagram of the part V in Fig. 4, Fig. 6 is a configuration diagram of an insulating ball, Fig. 7 is a configuration diagram of another insulating ball, and Fig. 8 is an enlarged diagram of a part where a lens moves.

This embodiment describes an image pickup unit 1 included in an image pickup apparatus to be used for an endoscope, in which an internal lens moves forward and backward as shown in Fig. 1 for a focus function or zooming/tele function. In the image pickup unit 1, an actuator 2 is provided on a mobile lens frame 4. A mobile lens 6 is attached to the mobile lens frame 4. The mobile lens 6 is an optical lens that moves forward and backward. The actuator 2 moves the mobile lens frame 4 forward and backward. In order to attach the actuator 2 included in an actuator apparatus to the mobile lens frame 4, a long cylindrical connecting pole 5 which is connected to a latching member for latching a wire, which will be described later is provided within the image pickup unit 1.

The mobile lens frame 4 is provided movable forward and backward along a shooting optical axis within a lens frame holding a front lens group and a substantially ring-shaped fixed lens frame 3 holding a lens group placed at the back. The outer circumferential part of the fixed lens frame 3 has a longitudinal groove that allows movement of the connecting pole 5. A movement butt ring 7 is fitted into the groove. The movement butt ring 7 is used for controlling the forward/backward movement of the connecting pole 5 and the mobile lens frame 4.

Next, the configuration of the actuator 2 attached to the image pickup unit 1 will be described.

A shape memory alloys (which will be called "SMA" hereinafter) wire 8 is fixed to the actuator 2 through the groove on the connecting pole 5 and folds back at the position of the connecting pole 5. The SMA wire 8 is a wire several tens microns in diameter, which contains SMA that contracts if heated and expands if cooled.

A first insulating tube 9 is externally inserted to the SMA wire 8 up to a middle part from the proximal end to the distal end. The first insulating tube 9 is partially inserted and fixed to the proximal end portion of a guide pipe 22.

A pressure spring 10 is held through the guide pipe 22 between the first insulating tube 9 and the connecting pole 5. The pressure spring 10 contains an elastic body, which is an urging unit, and presses the connecting pole 5 forward.

The guide pipe 22 has a guide groove 23, and the connecting pole 5 moves forward/backward within a notch in the guide groove 23.

The ends of the SMA wire 8 are caulked by a first caulking portion 16, which is a metallic ring in a tube shape. A first cable 17 is attached to the first caulking portion 16 by soldering. The first cable 17 applies current to the SMA wire 8. The circumference of the connecting part, not shown, between the first caulking portion 16 and the first cable 17 is reinforced and bonded by an adhesive, for example.

The first caulking portion 16 is attached at the distal end part of a flexible tube portion 230 beyond a bending portion 220 (at the position where the angle is manipulated by a bending piece 32) of an endoscope 200, as shown in Fig. 2.

The image pickup unit 1 is fixed to a rigid portion 210 of the endoscope 200. As shown in Fig. 3, the distal end surface of the rigid portion 210 of the endoscope 200 has an observation window 201, a cleaning nozzle 202, two illumination windows 201 and 203 and an opening 205 at predetermined positions. The observation window 201 is the distal end of the image pickup unit 1. The cleaning nozzle 202 cleans the observation window 201 by spraying fluid. The opening 205 is an opening of a channel through which a treatment instrument is inserted.

Referring back to Figs. 1 and 2, a first insulating tube 9 and a GND cable 27 are threaded through and fixed to a ring-shaped anchor 19. Then, a protective tube 20 is attached to the first insulating tube 9 and the GND cable 27 for covering the anchor 19, the first insulating tube 9 and a mobile insulating tube 26. The distal end side of the protective tube 20 is fixed to the first insulating tube 9 by tying with a string 21 such as a gut.

Next, a method for fixing the SMA wire 8 and the connecting pole 5 will be described.

The distal end part of the SMA wire 8 extends through a narrow second insulating tube 11, as shown in Fig. 5. In order to prevent the conduction between the SMA wire 8 and the connecting pole 5, the second insulating tube 11 is bonded and fixed to and through the connecting pole 5 (refer to Fig. 8).

The SMA wire 8 is wound once about and through an insulating ring 12, which is a latching member including a narrow fixing insulating tube (refer to Fig. 6). The insulating ring 12 is placed within an insulating ball 30, which is a ball formed by an adhesive. The insulating ball 30 is latched with and connected and fixed to the connecting pole 5 with an adhesive or the like.

As shown in Fig. 5, a GND SMA wire 28, which extends beyond the insulating ball 30 of the SMA wire 8 folds back through the inside of the mobile insulating tube 26 and a third insulating tube 25. In other words, the SMA wire 8 is configured to keep the electric insulation by the first insulating tube 9, the second insulating tube 11, the insulating ring 12, an insulating cap 13, an insulating pipe 15, the mobile insulating tube 26, the third insulating tube 25 and a second thermal contraction tube 24.

An end of the GND (ground) SMA wire 28 is caulked by a second caulking portion 29, as shown in Fig. 4. A GND (ground) cable 27 is soldered to a side of the second caulking portion 29.

Here, as described above, in the actuator 2 of the image pickup unit 1 of the present embodiment, the connecting position by soldering between the first caulking portion 16 fixed to the end of the SMA wire 8 and the lead line of he first cable 17 and the connecting position by soldering between the second caulking portion 29 fixed to the end of the GND SMA wire 28 and the lead line of the GND cable 27 are spaced apart by a predetermined distance in the longitudinal direction.

In other words, as shown in Fig. 2, the connecting position by soldering between the first caulking portion 16 fixed to the end of the SMA wire 8 and th lead line of the first cable 17 is different by a predetermined separating distance toward the proximal end from the connecting position by soldering between the second caulking portion 29 fixed to the end of the GND SMA wire 28 and the lead line of the GND cable 27.

More specifically, according to the present embodiment, the connecting position by soldering between the second caulking portion 29 fixed to the end of the GND SMA wire 28 and the lead line of the GND cable 27 exists around the boundary between the rigid portion 210 and the bending portion 220 of the endoscope 200. The connecting position by soldering between the first caulking portion 16 fixed to the end of the SMA wire 8 and the lead line of the first cable 17 exists near the distal end of the flexible tube portion 230 of the endoscope 200.

The dimensions are defined so as to satisfy t1<10α (refer to Fig. 6) where the diameter of the SMA wire 8 is α, and the length in the axial direction of the insulating ring 12 is t1.

Like the length t1 in Fig. 6, the length in the axial direction of the insulating ring 12 is sufficiently shorter than the diameter of the SMA wire 8, the insulating ring 12 is pulled by the SMA wire 8 and inclines. Thus, the SMA wire 8 passes through substantially the center of the insulating ball 30. Therefore, the insulating ball 30 is prevented from being caught by the guide pipe 22.

On the other hand, in a case where the dimensions are defined so as to satisfy t2>10α (longer than 10 times) where the diameter of the SMA wire 8 is α, and the length of the insulating ring 12 is t2 and if the length of the insulating ring 12 is longer than the diameter of the SMA wire 8, as shown in Fig. 7, the insulating ring 12 cannot rotate and is pressed by the repulsion of the SMA wire 8. Thus, the SMA wire 8 passes by the end of the insulating ball 30' (y1>y2). In this configuration, the SMA wire 8 causes offset with respect to the insulating ball 30, and the insulating ball 30 is caught by the guide pipe 22, which may cause poor sliding of the SMA wire 8.

Therefore, according to the present embodiment, the length t1 in the axial direction of the insulating ring 12 is defined to be shorter than 10 times of the diameter a of the SMA wire 8.

Next, an operation by the image pickup unit 1 of the present embodiment with the configuration described above will be described.

In order to drive the actuator 2 of the image pickup unit 1 by the endoscope 200 for a focus function or a zooming/tele function to a subject, current flows from a power supply included in a driving source, not shown, to the cable 17 based on a predetermined operation by an operating section, not shown, of the endoscope. Then, the current sequentially flows to the cable 17, the SMA wire 8, the GND SMA wire 28 and the GND cable 27, and the SMA wire 8 and the GND SMA wire 28 generate heat and contract.

Then, the mobile insulating tube 26 is pulled to move until the state of a mobile insulating tube 26', which is indicated by the broken line, is obtained. Here, the insulating ball 30 latched in front of the connecting pole 5 is pulled by the SMA wire 8 against the urging force of the pressure spring 10. Then, the mobile lens frame 4 is interlocked with the connecting pole 5 in the movement toward the proximal end and moves to the position of the mobile lens frame 14, which is indicated by the broken line.

Since the SMA wire 8 and the GND SMA wire 28 exhibit an identical contracting behavior, an undue load is not imposed on the distal end parts of the SMA wire 8 and GND SMA wire 28, which allows a stable behavior thereby.

Here, when the current flow to the cable 17 is stopped, the SMA wire 8 and GND SMA wire 28 are naturally cooled and return to the original lengths. At that time, the force by the pressure spring 10 pushes and moves the connecting pole 5 forward. With this, the mobile lens frame 14 moved toward the proximal end moves from the position indicated by the broken line to the position of the mobile lens frame 4.

In this way, according to the present embodiment, the SMA wire 8 folds back at the distal end, and the connecting position between the lead line of the cable 17 and the SMA wire 8 is spaced apart in the longitudinal axis direction to the proximal end side from the connecting position between the lead line of the GND cable 27 and the GND SMA wire 28. In addition, the SMA wire 8 is connected at the fine outside shape of the rear part of the image pickup unit 1. Therefore, the diameter of the rigid portion 210, which is the distal end portion of the endoscope 200, can be reduced.

Furthermore, the diameter of the distal end portion of the endoscope 200 can be reduced by not providing the connecting position between the lead line of the cable 17 and the SMA wire 8 at the distal end of the SMA wire 8. Since the SMA wire 8 has bad bondability, or so-called spreadability, the diameter being as narrow as several tens microns, the first caulking portion 16 is connected to the SMA wire 8, which prevents an increase of the size of the connecting part by soldering with the lead line of the cable 17.

### [Second Embodiment]

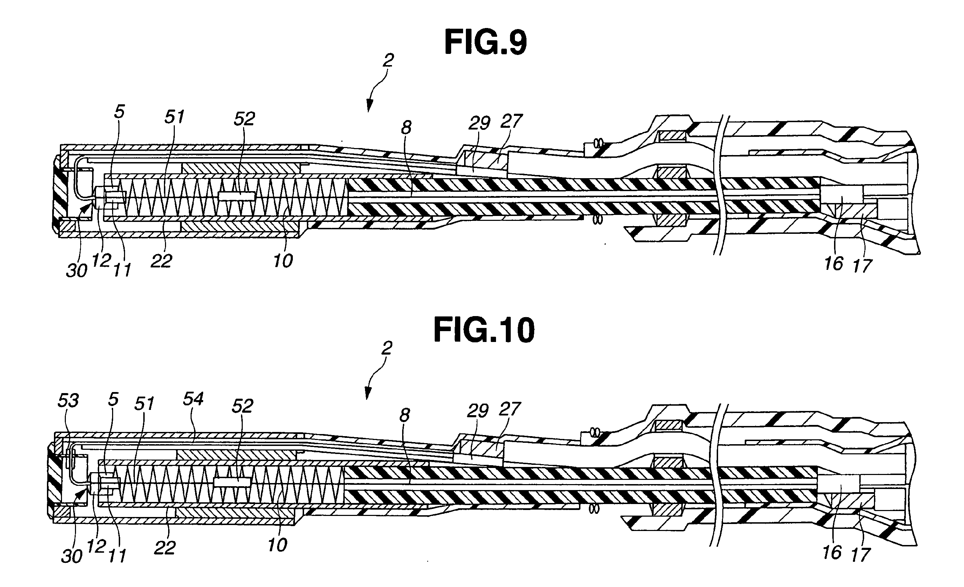

Next, with reference to Fig. 9, an actuator 2 of an image pickup unit 1 of a second embodiment will be described. The same reference numerals are given to the same components as those of the first embodiment, and the description thereon will be omitted. Only differences therebetween will be described. Fig. 9 relates to the second embodiment of the invention and is a section diagram corresponding to Fig. 4 of the first embodiment.

According to the present embodiment, instead of the GND SMA wire 28 in the first embodiment, a wire 51 is used which contains a conductive material such as stainless and steel. The proximal end of the wire 51 is caulked to the second caulking portion 29 and is electrically connected to the GND cable 27 by soldering. On the other hand, the other end of the wire 51 on the distal end side is caught and folds back at the insulating ring 12 (refer to Fig. 6), like the SMA wire 8 of the first embodiment, and the wire 51 extends through the insulating tube 11. The wire 51 and the SMA wire 8 are caulked and connected by a metallic wire caulking portion 52.

According to the present embodiment, the wire 51 and the SMA wire 8 are connected by the wire caulking portion 52 to be electrically connected within the guide pipe 22.

Also in this configuration, the current applied to the cable 17 subsequently flows into the first caulking portion 16, the SMA wire 8, the wire caulking portion 52, the wire 51, the second caulking portion 29 and the GND cable 27. Thus, according to the present embodiment, the heat generation or cooling to the normal temperature of the SMA wire 8 causes the mobile lens frame 4 interlocked with the connecting pole 5, to be pulled by the SMA wire 8 against the urging force of the pressure spring 10 and move toward the proximal end or to be pushed to move forward by the force of the pressure spring 10.

Therefore, like the first embodiment, the actuator 2 of the image pickup unit 1 can be driven by the endoscope 200 for a focus function or zooming/tele function to a subject.

The configuration of the actuator 2 of the present embodiment can also provide the same operational effects as those of the first embodiment as described above.

### [Third Embodiment]

Next, with reference to Fig. 10, an actuator 2 of an image pickup unit 1 of a third embodiment will be described. The same reference numerals are given to the same components as those of the first and second embodiments, and the description thereon will be omitted. Only differences therebetween will be described. Fig. 10 relates to the third embodiment of the invention and is a section diagram corresponding to Fig. 4 of the first embodiment.

The actuator 2 of the present embodiment has a configuration in which an SMA wire 54 is connected to the GND side of the wire 51 of the second embodiment. The SMA wire 54 of the present embodiment may be replaced by the GND SMA wire 28 of the first embodiment.

One end on the proximal end of the SMA wire 54 is caulked to the second caulking portion 29 and is electrically connected to the GND cable 27. The other end on the distal end of the SMA wire 54 is caulked and connected with the wire 51 by a metallic caulking portion 53. The other configuration is the same as that of the second embodiment.

Also in this configuration, the current applied to the cable 17 sequentially flows into the first caulking portion 16, the SMA wire 8, the wire caulking portion 52, the wire 51, the caulking portion 53, the SMA wire 54, the second caulking portion 29 and the GND cable 27.

Thus, according to the present embodiment, the heat generation or cooling to the normal temperature of the SMA wires 8 and 54 causes the mobile lens frame 4 interlocked with the connecting pole 5, to be pulled by the SMA wire 8, 54 against the urging force of the pressure spring 10 and move toward the proximal end or to be pushed to move forward by the force of the pressure spring 10.

Therefore, like the first embodiment, the actuator 2 of the image pickup unit 1 can be driven by the endoscope 200 for a focus function or zooming/tele function to a subject.

The configuration of the actuator 2 of the present embodiment can also provide the same operational effects as those of the first and second embodiments as described above.

### [Fourth Embodiment]

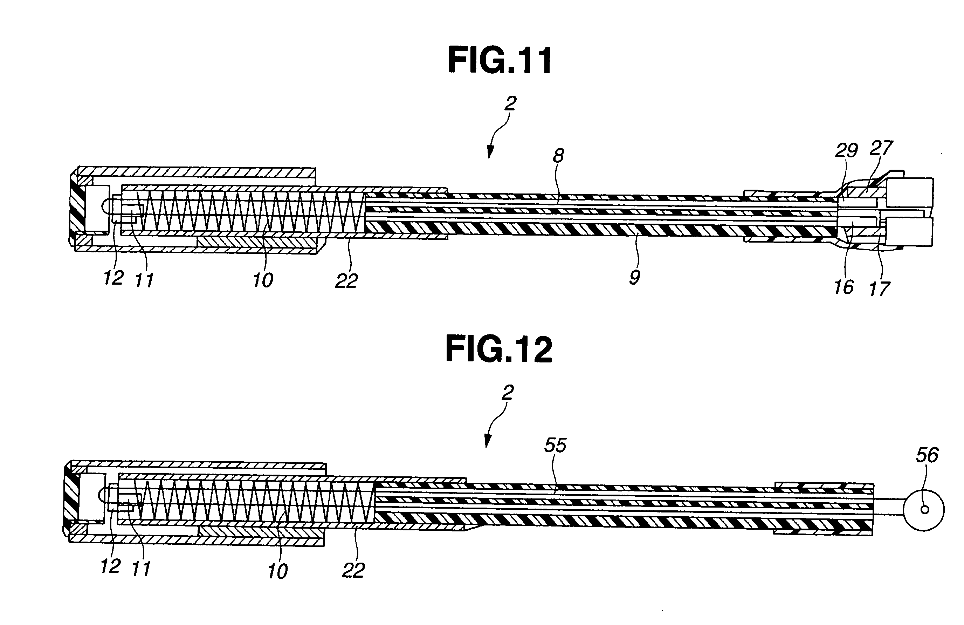

Next, with reference to Fig. 11, an actuator 2 of an image pickup unit 1 of a fourth embodiment will be described. Also in the following description, the same reference numerals are given to the same components as those of the first to third embodiments, and the description thereon will be omitted. Only differences therebetween will be described. Fig. 11 relates to the fourth embodiment of the invention and is a section diagram corresponding to Fig. 4 of the first embodiment.

According to the present embodiment, the insulating tube 9 may be a multilumen tube having two wire through holes, and both ends of the SMA wire 8 extend through the through holes from the distal end side.

More specifically, the SMA wire 8 folds back at the insulating ring 12 to have a loop shape on the distal end side of the guide pipe 22 fixed to the insulating tube 9. Then, both ends of the SMA wire 8 are caulked by the caulking portions 16 and 29 at the same position (distance) from the distal end.

The positions of the caulking portions 16 and 29 are connecting positions near the distal end of the flexible tube 230 of the endoscope 200 according to the first embodiment.

Both wires of the folded SMA wire 8 are fixed through the insulating tube 9 so as to pull the insulating ring 12 against the urging force of the pressure spring 10 by the heat generation by the passage of current.

The actuator 2 of the present embodiment having the configuration as described above can pull the insulating ring 12 with an amount of force substantially twice as that of the first embodiment. As a result, the actuator 2 can move the mobile lens frame 4 forward/backward instantly (in a short period of time) for a focus function or zooming/tele function to a subject.

### [Fifth Embodiment]

Next, with reference to Fig. 12, an actuator 2 of an image pickup unit 1 of a fifth embodiment will be described. Also in the following description, the same reference numerals are given to the same components as those of the first to fourth embodiments, and the description thereon will be omitted. Only differences therebetween will be described. Fig. 12 relates to the fifth embodiment of the invention and is a section diagram corresponding to Fig. 4 of the first embodiment.

In the actuator 2 of the present embodiment, the insulating ring 12 is pulled/loosened by a wire 55 simply of stainless or steel, for example, without using an SMA wire.

Both ends of the wire 55 of the present embodiment extend through the insulating tube 9 having two wire through holes, like the SMA wire 8 of the fourth embodiment, and the wire 55 folds back at the insulating ring 12 on the distal end side of the guide pipe 22. Both ends of the wire 55 are fixed to a motor head 56 attached to a motor (not shown) constituting a driving source.

In the actuator 2 having the configuration, the motor head 56 is driven to rotate by the motor, and the wire 55 is wound about the motor head 56. Thus, the insulating ring 12 moves backward against the urging force of the pressure spring 10. The reverse rotation of the motor returns the wire 55 wound about the motor head 56, and the insulating ring 12 moves forward by the urging force of the pressure spring 10.

The actuator 2 of the present embodiment having the configuration as described above can move the mobile lens frame 4 forward/backward instantly (in a short period of time) for a focus function or zooming/tele function to a subject, like the effects of the fourth embodiment described above. The actuator 2 is configured to prevent an increase in temperature by the heat generation of an SMA wire since an SMA wire is not used.

Having described the aspect in which the actuator 2 is attached to the mobile lens frame 4 to move a lens according to the embodiments above, the subject to be moved is not limited to a lens. The present invention is also applicable to the transportation of parts in a manufacturing line in a factory, for example.

Based on the descriptions above, according to the invention, the size of the actuator apparatus can be reduced, which can reduce the sizes and diameters of the distal end portions of an image pickup apparatus and endoscope.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An actuator apparatus comprising:
a mobile unit that is movable forward and backward;
an urging unit that urges the mobile unit in one direction; and
a wire fixed to the mobile unit,
wherein both ends of the wire are connected to a driving source that moves the mobile unit in the other direction against the urging force by the urging unit.

2. The actuator apparatus according to Claim 1,
wherein the urging unit includes an elastic body.

3. The actuator apparatus according to Claim 1 or 2,
wherein at least a part of the wire includes a shape memory alloys material; and
both ends of the wire are connected to a cable that supplies/receives electricity to/from a power supply.

4. The actuator apparatus according to any one of Claims 1 to 3,
wherein the positions of both ends of the wire connecting to the cable are different from each other.

5. An image pickup apparatus comprising:
an actuator apparatus having a mobile unit that is movable forward and backward, an urging unit that urges the mobile unit in one direction, and a wire fixed to the mobile unit, in which both ends of the wire are connected to a driving source that moves the mobile unit in the other direction against the urging force by the urging unit,
wherein:
the mobile unit has an optical lens; and
the wire is connected so as to fold back at a latching member connecting to the mobile unit.

6. The image pickup apparatus according to Claim 5,
wherein the urging unit includes an elastic body.

7. The image pickup apparatus according to Claim 5 or 6,
wherein at least a part of the wire includes a shape memory alloys material; and
both ends of the wire are connected to a cable that supplies/receives electricity to/from a power supply.

8. The image pickup apparatus according to any one of Claims 5 to 7,
wherein the positions of both ends of the wire connecting to the cable are different from each other.

9. An endoscopic apparatus comprising:
an actuator apparatus having a mobile unit that is movable forward and backward, an urging unit that urges the mobile unit in one direction, and a wire fixed to the mobile unit, in which both ends of the wire are connected to a driving source that moves the mobile unit in the other direction against the urging force by the urging unit; and
an image pickup apparatus in which the mobile unit has an optical lens, and the wire is connected so as to fold back at a latching member connecting to the mobile unit,
wherein one and the other ends of the wire are connected to the cable at the front and the rear portion of the bending portion, respectively.

10. The endoscopic apparatus according to Claim 9,
wherein the urging unit includes an elastic body.

11. The endoscopic apparatus according to Claim 9 or 10,
wherein at least a part of the wire includes a shape memory alloys material; and
both ends of the wire are connected to a cable that supplies/receives electricity to/from a power supply.

12. The endoscopic apparatus according to any one of Claims 9 to 11,
wherein the positions of both ends of the wire connecting to the cable are different from each other.
